# EUROPEAN PATENT APPLICATION

(11) **EP 3 449 915 A1**
(43) Date of publication of application: **06.03.2019**
(21) Application number: 18190961.5
(22) Date of filing: 27.08.2018
(51) Int. Cl.: A61K 31/05, A61K 31/352, A61K 9/68, A61P 17/00

(54) **COMPOSITION CONTAINING CANNABINOIDS FOR USE IN A METHOD FOR THE TREATMENT OF ATOPIC DERMATITIS**

(30) Priority: 28.08.2017 US 201762550880 P
(71) Applicant: Axim Biotechnologies, Inc., New York, New York 10022 (US)
(72) Inventor: Changoer, Lekhram, 2987 VD Ridderkerk (NL); Anastassov, George Evgeniyevich, New York, NY New York 10017 (US)
(74) Representative: Nederlandsch Octrooibureau

(57) **Abstract**

The current invention relates to a composition containing cannabinoids for use in a method for the treatment of atopic dermatitis, wherein said composition containing cannabinoids is applied topically onto skin areas in a subject present with atopic dermatitis. Typically, in the composition containing cannabinoids for use of the invention, the cannabinoids are cannabidiol and cannabigerol at a total weight percent of 3 to 20% of the composition. A method to treat the skin condition atopic dermatitis is also described in this invention. The method comprises topical application of a composition containing cannabinoids, specifically cannabidiol and cannabigerol at a concentration of 3 - 20% by weight of the composition. The method may further comprise administering chewing gum containing cannabidiol at 10 mg a piece. Cannabidiol and cannabigerol may be sourced naturally or synthetically.

## Description

### Field of the Invention

This invention concerns the field of treatment for skin conditions caused by alterations in the immune system. Various such skin conditions exist and various methods have been introduced for their treatment. The invention thus relates to a composition containing cannabinoids for use in a method for the treatment of atopic dermatitis, wherein said composition containing cannabinoids is applied topically onto skin areas in a subject present with atopic dermatitis.

### Background of the invention

Atopic dermatitis (AD), also known as eczema, is a type of inflammation of the skin. It results in itchy, red, swollen, and cracked skin. Clear fluid may extravasate from the affected skin, which often thickens over time. The condition typically starts in childhood with fluctuating severity over the years. AD is a chronic condition with sometimes similar appearance to psoriasis with red patches which can be thick, silvery and scaly. But the skin is thinner and less inflamed than in psoriasis.

The cause is unknown but believed to involve genetics, immune system dysfunction, environmental exposures, and altered permeability of the skin. About 30% of the people with atopic dermatitis have mutations in the gene for the production of filaggrin (*FLG*), which increase the risk for early onset of atopic dermatitis and developing asthma. Filaggrin plays an important role in keeping the skin surface slightly acidic; hence giving it anti-microbial effects. It breaks down into trans-urocanic acid, which keeps the pH low.

Evidence suggests that interleukin 4 (IL-4) is central in the pathogenesis of AD. Therefore, there is a rationale for targeting IL-4 with anti-IL-4 inhibitors. Other interleukins, such as IL-2 and IL-31 are also thought to play a role in atopic dermatitis. IL-2 therapy has been explored as a treatment for atopic dermatitis.

Currently, there is no cure for AD, and treatment is mostly similar to the treatment of psoriasis (vitamin D, corticosteroids and UV). That is to say, AD patients are treated according to standard treatments for psoriasis, including application of creams with steroids or vitamin D3, exposure to ultraviolet light (UV), and administering corticosteroids. The frequent application of topical corticosteroid creams often leads to skin which no longer responds to treatment. Also, this treatment does not work well in many patients. Vitamin D3 creams rarely are sufficient to improve the skin's condition. UV therapy implies risk of skin damage and of skin cancer.

### ABBREVIATIONS

AD: Atopic dermatitis
CB1: Cannabinoid receptors type 1
CB2: Cannabinoid receptors type 2
CBC: Cannabichromene
CBD: Cannabidiol
CBDV: Cannabidivarin
CBG: Cannabigerol
CBN: Cannabinol
eCB: Endocannabinoid
THC: Tetrahydrocannabinol

### SUMMARY OF THE INVENTION

A first aspect of the invention relates to a composition containing cannabinoids for use in a method for the treatment of atopic dermatitis, wherein said composition containing cannabinoids is applied topically onto skin areas in a subject present with atopic dermatitis.

Preferably, in the composition containing cannabinoids for use according the invention, the cannabinoids are cannabidiol and cannabigerol at a total weight percent of 3 to 20% of the composition. Preferred are compositions containing cannabinoids for use of the invention, wherein the cannabinoids are cannabidiol and cannabigerol at a total weight percent of over 3% by weight of the composition, such as at least 15% by weight of the composition, or about 15% by weight of the composition. Preferably, the composition containing cannabinoids for use of the invention comprises more than 2% cannabigerol by weight of the composition and more than 1% cannabidiol by weight of the composition, wherein the cannabigerol is present at a twice to three times the weight of cannabidiol, preferably more than 3% cannabigerol and more than 1-1.5% cannabidiol, such as about 10% cannabigerol and about 5% cannabidiol by weight of the composition, according to the invention. Typically, in the composition containing cannabinoids for use according to the invention the cannabidiol is present at a total weight percent of 3% of the composition and cannabigerol is present at a total weight percent of 15% of the composition. In an embodiment, the composition containing cannabinoids for use according to the invention comprises cannabidiol and cannabigerol, wherein the cannabigerol is present at twice to three times the weight of cannabidiol.

The cannabis plant has many naturally occurring substances that are of great interest in the fields of science and medicine. Isolated compounds from the cannabis plant include Δ⁹-tetrahydrocannabinol (THC), cannabidiol (CBD), cannabichromene (CBC), cannabigerol (CBG), cannabinol (CBN), cannabidivarin (CBDV), among many other compounds. While THC has psychoactive effects, CBD, CBC, CBG, and CBDV do not. Isolated alkaloid compounds from the cannabis plant are called cannabinoids. There are a total of one hundred and forty one (141) cannabinoids that have been isolated from the cannabis plant. Many researchers have confirmed the medicinal value of cannabinoids. Cannabinoids have been investigated for possible treatment of seizures, nausea, vomiting, lack of appetite, pain, arthritis, inflammation, and other conditions.

Cannabinoids can be isolated by extraction or cold pressing from cannabis plants. Plants in the cannabis genus include *Cannabis sativa, Cannabis ruderalis,* and *Cannabis indica.* These plants are natural sources of cannabinoids. Cannabinoids are also available in synthetic forms. Methods to synthesize cannabinoids in lab setting were discovered and are still currently practiced. Synthetic cannabinoids are more targeted, in that the synthetic compound usually comes isolated without other cannabinoids and/or other compounds mixed in.

Nabilone (racemic(6aR,10aR)-1-hydroxy-6,6-dimethyl-3-(2-methyloctan-2-yl)-7,8,10,10a-tetrahydro-6H-benzo[c]chromen-9(6aH)-one), a synthetic cannabinoid, is believed to have fewer undesired side effects than THC. Nabilone mimics the chemical compound structure of THC. THC also exists in synthetic form under the name Dronabinol ((-)-(6aR,10aR)-6,6,9-trimythel-3-pentyl-6a,7,8,10a-tetrahydro-6H-benzo[c]chromen-1-ol)). The U.S. Food and Drug Administration approved nabilone and dronabinol for treatment of chemotherapy-induced nausea and vomiting and later for cachexia due to HIV/ AIDS. In the United States, nabilone is marketed under the name Cesamet® and dronabinol under the name Marinol®. There are also generic versions of the drugs available on the market.

The IUPAC nomenclature of THC is (-)-(6aR,10aR)-6,6,9-trimethyl-3-pentyl-6a,7,8,10a-tetrahydro-6H-benzo[c]chromen-1-ol. CBD's IUPAC nomenclature is 2-((lS,6S)-3-methyl-6-(prop-1-en-2-yl)cyclo-hex-2-enyl)-5-pentylbenzene-1,3-diol). CBC has the IUPAC nomenclature of 2-methyl-2-(4-methylpent-3-enyl)-7pentyl-5-chromenol. CBG has the IUPAC nomenclature of 2-[(2E)-3,7-dimethylocta-2,6-dienyl]-5-pentyl-benzene-1,3-diol. These are among the most prominent compounds in the family of compounds extracted from the cannabis plant referred to as cannabinoids.

Cannabidiol is a major phytocannabinoid, accounting for up to 40% of the plant's extract. CBD is a CB-1 receptor antagonist, while THC is a CB-1 receptor agonist. A 2010 research found that cannabis strains with higher concentration of CBD did not produce the short-term memory impairment normally seen in high THC cannabis strain, a characteristic attributed to the CB-1 receptor antagonist nature of CBD. CBD is considered to have a wider scope of medical applications than THC.

Because it is a relatively unknown cannabinoid, cannabigerol (CBG) remains understudied and its effects are only just starting to become elucidated. CBG is a non-psychoactive cannabinoid found in the cannabis plant. All cannabinoids in the early stage of the cannabis plant's life begin as CBG. CBG is found in higher concentrations in hemp plants as opposed to marijuana plants, which are grown to have higher concentrations of tetrahydrocannabinol (THC). CBG has been found to act as a high affinity α₂-adrenergic receptor agonist, a moderate affinity to 5-HT_{1A} receptor antagonist, and a low affinity CB₁ receptor antagonist. It binds with the CB₂ receptor, but it is currently unknown whether it acts as an agonist or antagonist.

Typically, for the composition containing cannabinoids for use according to the invention, cannabinoids in said composition are sourced naturally. In addition or alternatively, for the composition containing cannabinoids for use according to the invention, cannabinoids in said composition are synthetic, according to the invention.

Preferred is a composition containing cannabinoids for use according to the invention, wherein cannabinoids in the composition are in powder form and/or are in crystalline form, prior to incorporation into the composition.

In an embodiment, the cannabinoids comprised by the composition containing cannabinoids for use according to the invention are microencapsulated. In an embodiment said cannabinoids in the composition containing cannabinoids for use according to the invention are nanoencapsulated with particle sizes of 20 to 40 nanometers.

Preferably, the composition containing cannabinoids for use according to the invention further comprises hyaluronic acid derivatives. Typically, such a hyaluronic acid derivative in the composition containing cannabinoids for use of the invention is sodium oleyl hyaluronate.

In one embodiment, the composition containing cannabinoids for use according to the invention comprises at least one of omega-3 or omega-6 fatty acids. Thus, the composition containing cannabinoids for use according to the invention, further comprising at least one of omega-3 or omega-6 fatty acids and comprising hyaluronic acid derivatives such as sodium oleyl hyaluronate, are also part of the invention.

Typical compositions containing cannabinoids for use according to the invention further comprises plant extracts. Preferred plant extracts comprised by the composition containing cannabinoids for use of the invention are neem, curcuma longa, rubia cardifolia, or wrightia tinctorial extract, according to the invention. Thus, typically, the composition containing cannabinoids for use of the invention comprises at least one of omega-3 or omega-6 fatty acids, and/or comprises hyaluronic acid derivatives such as sodium oleyl hyaluronate, and/or comprises plant extracts such as one or more of neem, curcuma longa, rubia cardifolia, or wrightia tinctorial extract, according to the invention.

Preferably, the composition containing cannabinoids for use according to the invention further comprises cacao butter. Thus, typically, the composition containing cannabinoids for use of the invention comprises cacao butter, and/or comprises at least one of omega-3 or omega-6 fatty acids, and/or comprises hyaluronic acid derivatives such as sodium oleyl hyaluronate, and/or comprises plant extracts such as one or more of neem, curcuma longa, rubia cardifolia, or wrightia tinctorial extract, according to the invention.

According to the invention, it is preferred that the composition containing cannabinoids for use according to the invention is in a hydro gel form, a liquid form, a spray form, a powder form, or an ointment form.

In some embodiments, the composition containing cannabinoids for use according to the invention is administered to the subject suffering from AD before, after and/or concomitantly with the oral administration of chewing gum containing cannabidiol to said subject. Typically, the composition containing cannabinoids for use of the invention is administered twice daily, and typically, the chewing gum containing cannabidiol is administered thrice daily to the subject, i.e. the patient suffering from AD.

In an embodiment, the composition containing cannabinoids for use according to the invention is administered to a patient suffering from AD, said patient also receiving the chewing gum containing cannabidiol, wherein the chewing gum contains 10 milligrams of cannabidiol in each piece. Typically, for the composition containing cannabinoids for use according to the invention, the chewing gum is administered 3 times per day.

Preferred is the composition containing cannabinoids for use according to the invention, wherein said composition is administered to the subject twice daily.

The present invention also provides a method to treat atopic dermatitis using compositions containing cannabinoids, namely cannabidiol and cannabigerol. Cannabinoids are present at 3 to 20% by weight of the total composition. The composition may be gel, liquid, spray, powder, or ointment form. Cannabinoids may be sourced naturally or synthetically and may be nano-encapsulated or micro-encapsulated. This method to treat atopic dermatitis may further comprise oral administration of chewing gums containing cannabidiol concomitantly with the topical application.

A second aspect of the invention relates to a method to treat atopic dermatitis, the method comprising topical application of a composition containing cannabinoids onto skin areas in a subject present with atopic dermatitis.

Preferred is the method of the invention, wherein the cannabinoids are cannabidiol and cannabigerol at a total weight percent of 3 to 20% of the composition. Typically, for the method of the invention, the cannabidiol is present at a total weight percent of 3% of the composition and cannabigerol is present at a total weight percent of 15% of the composition. Particularly preferred is the method of the invention, wherein the cannabigerol is present at a twice to three times the weight of cannabidiol.

In an embodiment, for the method of the invention, the cannabinoids in the composition are sourced naturally. In another embodiment, for the method of the invention, the cannabinoids in the composition are synthetic. Preferably, for the method of the invention, the cannabinoids in the composition are in powder form prior to incorporation into the composition. Preferably, for the method of the invention, the cannabinoids in the composition are in crystalline form prior to incorporation into the composition.

Typically, in the method of the invention, the cannabinoids in the composition are microencapsulated. Preferred is the method according to the invention, wherein cannabinoids in the composition are nanoencapsulated with particle sizes of 20 to 40 nanometers.

In embodiments, for the method of the invention, the composition further comprises hyaluronic acid derivatives. Preferably, in the method of the invention, said hyaluronic acid derivatives is sodium oleyl hyaluronate.

It is preferred that in the method of the invention the composition further comprises at least one of omega-3 or omega-6 fatty acids. Also preferred is the method of the invention, wherein the composition further comprises plant extracts. Typically, these plant extracts are neem, curcuma longa, rubia cardifolia, or wrightia tinctorial extract.

In the method according to the invention, the composition applied in said method preferably further comprises cacao butter.

Particularly preferred is the method according to the invention, wherein the composition that is applied in said method is in a hydro gel form, a liquid form, a spray form, a powder form, or an ointment form.

Preferably, the method of the invention further comprises oral administration of chewing gum containing cannabidiol on the subject. In an embodiment, for the method of the invention, the chewing gum contains 10 milligrams of cannabidiol in each piece. It is preferred that for the method of the invention, the chewing gum is administered 3 times per day.

### DETAILED DESCRIPTION OF CERTAIN INVENTIVE EMBODIMENTS

Embodiments of this application relate to compositions containing cannabinoids for use in a method for the treatment of AD, wherein said composition containing cannabinoids is applied topically onto skin areas in a subject present with AD. Further, embodiments of this application relate to compositions comprising cannabinoids for use in a method for the treatment of AD, wherein said composition comprising cannabinoids is applied topically onto skin areas in a subject present with AD. The compositions comprising or containing cannabinoids for use in a method for the treatment of AD in particular comprise compositions comprising CBD and CBG. In addition, for the compositions comprising or containing cannabinoids for use in a method for the treatment of AD, said compositions are administered to a subject suffering from AD, to whom also chewing gum containing CBD is administered orally for trans-oral-mucosal delivery of cannabidiol (CBD). Further embodiments of this application relate to methods to treat the skin condition atopic dermatitis caused by altered immune system response. The methods presented concerns using topical application compositions containing cannabinoids, in particular CBD and CBG, to be applied to the subject's skin area, and chewing gum containing CBD administered orally for trans-oral-mucosal delivery of cannabidiol (CBD).

In embodiments, the composition for use in a method for the treatment of AD may be a composition such as an oil, an ointment, a cream, or a powder containing cannabinoids, namely CBD and CBG, while other cannabinoids may be present. In embodiments, the medicament used in this method of treatment may be a composition such as an oil, an ointment, a cream, or a powder containing cannabinoids, namely CBD and CBG, while other cannabinoids may be present. Cannabinoid oil may be from cannabis or hemp extraction and decarboxylated to reach desired cannabinoid concentrations. CBG may be present at twice to three times the amount of CBD by weight in this composition for use in the method for the treatment of AD and in this composition applied in the method of the invention.

In embodiments, cannabinoid compositions used in this composition for use in a method for the treatment of AD according to the invention and in this method of the invention may contain CBD/CBG oil at 3 - 20% by weight. The percentages given may include both cannabinoids, for example the total weight percentage of CBD/CBG in the composition is 3% and 15%, respectively. CBG may be present at twice to three times the amount of CBD in the same composition. Other cannabinoids may be present at lower concentration, such as lower than 1% by weight. It is contemplated that CBD/CBG composition at 5 - 20% by weight percent of the total composition may be used in this composition for use in a method for the treatment of AD according to embodiments and in this treatment method according to embodiments.

Alternatively, cannabinoids may also be incorporated into the composition for use in a method for the treatment of AD according to the invention and into the composition applied in the method of the invention from crystalline and/or powder form. Cannabinoids used in these embodiments must be at a high purity, such as 99% purity, but could be lower or higher. Cannabinoids concentration in the composition used in this invention may be at 0.5 - 6% of the total composition by weight.

The composition for use in a method for the treatment of AD according to the invention and the medicament applied in the method of the invention may also be a composition containing cannabinoids with additional components formulated into a composition for topical application. The cannabinoids in the formulation may be in nano-encapsulated form and the size of the particles is between 20 and 40 nm. The cannabinoids in these compositions may also be microencapsulated. Cannabinoids may be sourced naturally or synthetically.

In embodiments, the composition for use in a method for the treatment of AD according to the invention and the composition used in this method of the invention may further comprised hyaluronic acid (HA) derivatives, including but not limited to sodium oleyl hyaluronate, tocopherol, omega-3, omega-6. Other plant extract ingredients may be present in this composition for use in a method for the treatment of AD according to the invention and in this composition used in the method of the invention, such as neen, curcuma longa, rubia cardifolia, wrightia tinctorial extracts, among other plant extracts. The composition may further comprise cacao butter, wherein cannabinoids are incorporated into cacao butter prior to being synthesized into the composition. Other components in this composition according to embodiments may include omega-3 and/or omega-6 fatty acids. Cannabinoids may be combined with omega-3 and/or omega-6 fatty acid.

The composition for use in a method for the treatment of AD according to the invention and the composition used in the method of the invention may further comprise other ingredients to effectuate the form in which the composition may be prior to usage. The composition may be prepared into a cream, an ointment, a gel, a hydro gel, a spray, a powder, or other composition form suitable for topical application.

Additional medicaments used in this study were chewing gums containing CBD at 10 mg a piece. Chewing gums may be consumed by mastication, upon which CBD is released and absorbed through the oral mucosa in a controlled fashion.

### Study Design

The therapeutic effect of a combination of the two non-psychotropic cannabinoids Cannabigerol (CBG) and Cannabidiol (CBD) in hempseed oil in a mass ratio of 2:1 and a combinational therapy with Canchew Hemp CBD gum (10 mg CBD/50 mg Hempoil) in patients with atopic dermatitis (AD) was tested.

Materials: 0% CBG/CBD, 3% CBG/CBD, 15% CBG/CBD in hempseed oil ointments were supplied by AXIM Biotechnologies. The CBG/CBD strains were obtained from the company Ecohemp SRL (Dr. Grassi; Italy).

Three (3) subjects with present atopic dermatitis who had received no therapy for at least six (6) weeks prior to the study were selected. Two (2) subjects received treatment by topical application of CBD/CBG oil, and one (1) subject received treatment by topical application of CBD/CBG oil and oral mastication of chewing gums containing CBD. The study was conducted on lesions present on the subjects' arms. Control was by means of lesions of similar size and severity on the subjects' other arm.

Cannabinoid oil used in this study is CBD/CBG oil at 3% by weight and CBD/CBG oil at 15% by weight. The percentages given may include both cannabinoids, such that the total weight percentage of CBD/CBG in the oil is 3% or 15%. Other cannabinoids may be present at lower concentration, such as lower than 1% by weight.

Chewing gum containing CBD at 10 mg was provided for mastication three (3) times a day, concurrently with topical application of CBD/CBG oil.

Each subject was evaluated for present skin lesions due to atopic dermatitis for choosing lesions for this study. On the left arm of each subject, two skin lesions at least twenty (20) centimeters away from each other were selected for treatment. Two (2) corresponding skin lesions on the right arm were selected for control.

Each lesion was assessed with an adapted version of the severity scoring of Atopic Dermatitis Severity Index (SCORAD). The following symptoms were scored: erythema (redness), desquamation (scaling infiltration), induration (thickness), excoriations (scratch marks), lichenification (skin thickening). Each score was graded as follows: none = 0, mild = 1, moderate = 2, severe = 3.

The study was conducted by topical application of CBD/CBG oil to lesions present on the subject's skin. CBD/CBG oil may be applied as a thin layer on the subject's skin. One lesion received on the left arm received topical application of CBD/CBG oil at 15% by weight of the total composition, the other lesion of the left arm received topical application of CBD/CBG oil at 3% by weight of the total composition. In these oils, CBG is present at twice the amount of CBD by weight. Two lesions on the right arm were selected and received placebo (0% CBD/CBG oil) to serve as controls. Chewing gums were given at 3 pieces per day (10 mg of CBD in each piece) and were consumed by mastication for a minimum of 30 minutes.

### Drug Treatment Procedure and Administration

Subjects received treatment twice daily for six (6) weeks as follows:
Each of the first two (2) subjects received topical application of a thin layer of 3% CBG/CBD oil on the upper lesion and 15% CBG/CBD oil on the lower lesion of the left arm and placebo (0% CBG/CBD oil) on the two lesions of the right arm. The treatment was given twice daily.

A third subject subjects received topical application of a thin layer of 3% CBG/CBD oil on the upper lesion and 15% CBG/CBD oil on the lower lesion of the left arm and placebo (0% CBG/CBD oil) on the two lesions of the right arm and 3 pieces of chewing gum containing CBD at 10 mg, which were consumed daily by mastication.

Scoring of the lesions was by an adapted version of Atopic Dermatitis Severity Index (SCORAD), which is Table 1 as follows:

**Table 1**

| **Symptom** | **Grading** |
|---|---|
| Erythemda (redness) | |
| Desquamation (scaling) | |
| Infiltration, induration (thickness) | |
| Excoriations (scratch marks) | |
| Lichenification (skin thickening) | |

Grading was given by score from 0 - 3. Each score was graded as follows: none = 0, mild = 1, moderate = 2, severe = 3.

At the endpoint of the study, results were the difference as percentage of improvement between the left and right corresponding lesions. Lesions on the right arms are control samples. The following was the result of the study as described herein.

**Table 2**

| **Condition** | **Subject** | **% Improvement of 3% CBD/CBG oil** | **% Improvement of 15% CBD/CBG oil** | **% Improvement of 15% CBD/CBG oil + CBD chewing gum** |
|---|---|---|---|---|
| Atopic dermatitis | Subject 1 | 0 | 22 | n/a |
| Atopic dermatitis | Subject 2 | 0 | 11 | n/a |
| Atopic dermatitis | Subject 3 | 0 | n/a | 33 |

### Discussion

Treatment by 3% CBG/CBD oil treatment showed no improvement on the lesions. The 15% CBG/CBD oil treatment showed 11% and 22% improvement on the two (2) subjects treated without CBD chewing gum. The 15% CBG/CBD oil treatment on the third subject treated with 3 pieces of CBD chewing gum daily showed a 33% improvement. The improvement was intra-specimen, where the subjects' other lesions served as their own controls.

Due the systemic penetration of the active ingredients (CBG/CBD), an overall improvement may have occurred for both the studied lesions. This may have negatively influenced the perceived effectiveness of the treatment, since CBD/CBG at a lower dose may not have penetrated the skin very well. CBG was dosed twice as much as CBD, and as such might counteract some unintended actions of CBD, just like CBD is thought to counteract the cognitive impairment caused by tetrahydrocannabinol (THC).

Activation of peripheral CB1 receptors contributes to hemorrhagic and endotoxin-induced hypotension. Without wishing to be bound by theory, both CBG and CBD act as CB1 antagonists, which might suggest a possible mechanism that explains a reduction in redness in skin lesions through reduction of vasodilatation. This is supported by the finding that cannabinoids can inhibit inflammatory cytokines and angiogenic growth factors such as hypoxia inducible factor-1 α (HIF-1 α), vascular endothelial growth factor (VEGF), matrix metalo-proteinases (MMPs), basic fibroblast growth factor (bFGF), Angiopoietin-2, interleukin-8 (IL-8), IL-17, and IL-2 as well as cellular adhesion molecule 1 (ICAM-1) and vascular cell adhesion molecule-1 (VCAM-1) both in vivo and in vitro. Contrary to these findings it seems that CBD induced CB1 activation causes endothelium-dependent vasorelaxation of human mesenteric arteries.

Without wishing to be bound by theory, the results from this study indicate a possible Thl/Th2 rebalancing mechanism. Despite the fact that only two concentrations have been used, there seems to be a clear dose-response effect, whereby higher doses (15% CBD/CBG oil) are effective where lower doses (3% CBD/CBG oil) do not show any effect. Factors influencing the dose effect might be a limited sufficient skin penetration, since skin penetration of cannabinoids may be poor.

The results present evidence for a possible synergistic role of CBD and CBG in dermatological conditions such as atopic dermatitis. Restoring the Thl/Th2 balance is thought to be the key mechanism of action, with a possible additional direct inhibiting effect of CBG on hyper proliferation of skin cells (scaling).

All references, including publications, patent applications, and patents cited herein are hereby incorporated by reference to the same extent as if each reference were individually and specifically indicated to be incorporated by reference and were set forth in its entirety herein.

It will be readily apparent to those skilled in the art that a number of modifications and changes may be made without departing from the spirit and the scope of the present invention. It is to be understood that any ranges, ratios, and range of ratios that can be derived from any of the data disclosed herein represent further embodiments of the present disclosure and are included as part of the disclosure as though they were explicitly set forth. This includes ranges that can be formed that do or do not include a finite upper and/or lower boundary. Accordingly, a person of ordinary skill in the art will appreciate that such values are unambiguously derivative from the data presented herein.

## Claims

1. Composition containing cannabinoids for use in a method for the treatment of atopic dermatitis, wherein said composition containing cannabinoids is applied topically onto skin areas in a subject present with atopic dermatitis.

2. Composition containing cannabinoids for use according to claim 1, wherein the cannabinoids are cannabidiol and cannabigerol at a total weight percent of 3 to 20% of the composition.

3. Composition containing cannabinoids for use according to claim 1 or 2, wherein the cannabidiol is present at a total weight percent of 3% of the composition and cannabigerol is present at a total weight percent of 15% of the composition.

4. Composition containing cannabinoids for use according to any one of the preceding claims, wherein the cannabigerol is present at twice to three times the weight of cannabidiol.

5. Composition containing cannabinoids for use according to any one of the preceding claims, wherein cannabinoids in the composition are sourced naturally or wherein cannabinoids in the composition are synthetic.

6. Composition containing cannabinoids for use according to claim 1, wherein cannabinoids in the composition are in powder form and/or are in crystalline form, prior to incorporation into the composition.

7. Composition containing cannabinoids for use according to any one of the preceding claims, wherein cannabinoids in the composition are microencapsulated or wherein cannabinoids in the composition are nanoencapsulated with particle sizes of 20 to 40 nanometers.

8. Composition containing cannabinoids for use according to any one of the preceding claims, wherein the composition further comprises hyaluronic acid derivatives.

9. Composition containing cannabinoids for use according to claim 8, wherein the hyaluronic acid derivatives is sodium oleyl hyaluronate.

10. Composition containing cannabinoids for use according to any one of the preceding claims, wherein the composition further comprises at least one of omega-3 or omega-6 fatty acids.

11. Composition containing cannabinoids for use according to any one of the preceding claims, wherein the composition further comprises plant extracts.

12. Composition containing cannabinoids for use according to claim 11, wherein the plant extracts are neem, curcuma longa, rubia cardifolia, or wrightia tinctorial extract.

13. Composition containing cannabinoids for use according to any one of the preceding claims, wherein the composition further comprises cacao butter.

14. Composition containing cannabinoids for use according to any one of the preceding claims, wherein the composition is in a hydro gel form, a liquid form, a spray form, a powder form, or an ointment form.

15. Composition containing cannabinoids for use according to any one of the preceding claims, wherein said composition containing cannabinoids is administered to the subject before, after and/or concomitantly with the oral administration of chewing gum containing cannabidiol to said subject.

16. Composition containing cannabinoids for use according to claim 15, wherein the chewing gum contains 10 milligrams of cannabidiol in each piece.

17. Composition containing cannabinoids for use according to claim 14 or 15, wherein the chewing gum is administered 3 times per day.

18. Composition containing cannabinoids for use according to any one of the preceding claims, wherein said composition is administered to the subject twice daily.
